# EUROPEAN PATENT APPLICATION

(11) **EP 1 256 572 A1**
(43) Date of publication of application: **13.11.2002**
(21) Application number: 01870100.3
(22) Date of filing: 10.05.2001
(51) Int. Cl.: C07D 205/08

(54) **Process for the deprotection of N-substituted azetidones**

(71) Applicant: Tessenderlo Chemie S.A., 1050 Bruxelles (BE)
(72) Inventor: Ceresiat, Marcel Maurice Ghislain, 1490 Court Saint Etienne (BE); Belmans, Marc, 3980 Tessenderlo (BE); Marchand-Brynaert, Jaqueline, 6001 Marcinelle (BE); Laurent, Mathieu, 1348 Louvain-la-Neuve (BE)
(74) Representative: Brants, Johan Philippe Emile

(57) **Abstract**

The present invention relates to a method for the preparation of a compound of the general formula (I) wherein
R¹ and R² each represent independently hydrogen, halogen, hydroxy, thiol, nitro, amino, carboxy, amino derivative, oxy derivative, thio derivative, alkyl, alkenyl, alkynyl, aryl, oxy derivative, amino derivative, thiol derivative, acyl derivative, acyloxy derivative, ester, amido, ether, arylalkyl, heterocycle or Me(OR')CH wherein R' is hydrogen or R^{a}R^{b}R^{c}Si wherein R^{a}, R^{b} and R^{c} is independently chosen from the group consisting of alkyl, alkenyl, alkynyl, aryl or a heterocycle;
R³ represents a protecting group such as alkyl, alkenyl, alkynyl, or arylalkyl;
wherein this method comprises the reaction of a compound of the general formula (II) in the presence of a halogen-radical source, water and a proton donor suitable as a catalyst.

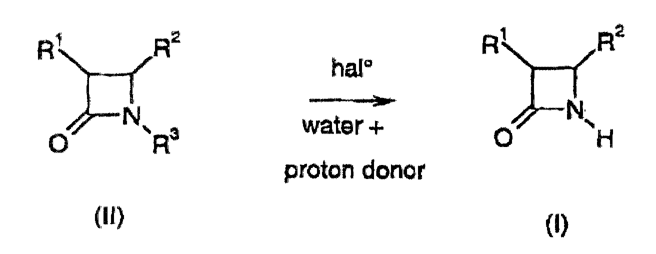

## Description

### FIELD OF THE INVENTION

This invention concerns an improvement in the process for the preparation of azetidones. Azetidones are useful intermediates for preparing carbapenem and penem type antibiotics.

### BACKGROUND

The present invention relates to an improvement in the process for the preparation of a compound which has the following general formula (I-A) wherein R' is hydrogen or R^{a}R^{b}R^{c}Si wherein R^{a}, R^{b} and R^{c} is independently methyl, ethyl, *i*-Pr, *t*-butyl or phenyl; and R" is hydrogen, alkyl, substituted alkyl, preferably t-butyl, phenyl, substituted phenyl, O-R^{d} or S-R^{d} wherein R^{d} is C₁₋₆ alkyl, substituted alkyl, phenyl or substituted phenyl.

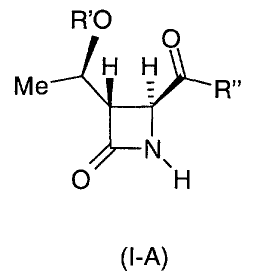

Compound of said general formula (I-A) may be prepared by a series of reactions as described hereafter.

First, L-threonine is converted to (2R,3R)-cis-2,3-epoxybutanoic acid as described in J. Chrom. A, 832:259-264, 1999. The (2R,3R)-cis-2,3-epoxybutanoic acid is next converted to an epoxyamide as described in Tetrahedron 56:3209-3217, 2000. In the following step, said epoxyamide is converted by ring closure to an azetidinone with a nitrogen-protecting group.

This nitrogen-protecting group should next be removed. Two methods are currently used to remove said nitrogen-protecting group. (A. H. Berks, Tetrahedron, 52, 331-375(1996))

A first method uses ceric ammonium nitrate (CAN) as disclosed in Kronenthal et al., J. Org. Chem., 47, 2765 (1982). This method was found to be useful when said nitrogen-protecting group consists of para-methoxyphenyl. (S. Hanessian et al., J.Am.Chem.Soc., 107, 1438-1439 (1985)).

The second method also consists of oxydative cleavage. For instance, in case the nitrogen-protecting group is 2,4-dimethoxybenzyl, said nitrogen-protecting group may be removed by the use of potassium persulfate as described in Y. Kita et al., Chem. Pharm. Bull., 40, 1733-1736 (1992). In case the nitrogen-protecting group consists of di(para-methoxyphenyl)methyl, the oxydative cleavage may be done by the use of sodium persulfate as disclosed in Y. Ito et al., Tetrahedron, 45, 5767-5790 (1989), or CAN as disclosed in M. Sunagawa et al., Chem. Pharm. Bull., 39, 1931-1938 (1991).

However, these deprotection processes as described above are quite difficult and expensive to conduct. This is particularly the case when said nitrogen-protecting group consists of a para-methoxyphenyl (anisyl) group.

It is clear that a new process for removing said nitrogen protecting group not having the disadvantages as mentioned above, not requiring a quantitative amount of metal (Ce IV) and still relatively easy to carry out, would be extremely desirable.

### DESCRIPTION

According to a first aspect, the present invention relates to a method for the preparation of a compound of the general formula (I) wherein
R¹ and R² each represent independently hydrogen, halogen, hydroxy, thiol, nitro, amino, carboxy, amino derivative, oxy derivative, thio derivative, alkyl, alkenyl, alkynyl, aryl, oxy derivative, amino derivative, thiol derivative, acyl derivative, acyloxy derivative, ester, amido, ether, arylalkyl, heterocycle or Me(OR')CH wherein R' is hydrogen or R^{a}R^{b}R^{c}Si
wherein R^{a}, R^{b} and R^{c} represent independently alkyl, alkenyl, alkynyl, aryl or a heterocycle, each of said radicals optionally substituted;
R³ represents a protecting group such as alkyl, alkenyl, alkynyl or arylalkyl, each optionally substituted;
wherein this method comprises the reaction of a compound of the general formula (II) in the presence of a halogen radical source, water and a proton donor as the catalyst, according to the following ***scheme 1.***

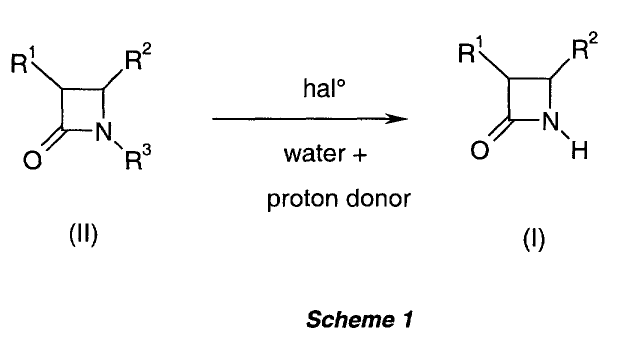

In the compounds and radicals as described above the term "alkyl" includes saturated hydrocarbon radicals having straight, branched or cyclic moieties or combinations thereof and contains 1-20 carbon atoms, preferably 1-10 carbon atoms and more preferably 1-5 carbon atoms. The alkyl group may optionally be substituted by 1 to 5 substituents independently selected from the group consisting of halogen, hydroxy, oxy derivatives, thiol, thio derivatives, amino, amino derivatives, nitro, acyl derivatives, acyloxy derivatives, carboxy, ester, ether, amido, heterocycle. Preferred alkyl groups are methyl, ethyl, propyl, isopropyl, butyl, iso- or *t*-butyl.

The term "alkenyl" refers to an univalent C₂ to C₁₂ and preferably C₂ to C₆ straight or branched, hydrocarbon with at least one double bond, optionally substituted with any suitable group, including but not limited to one or more moieties selected from lower alkyl or other groups as described above for the alkyl groups. Non-limiting examples are ethenyl, 1-propenyl, 2-butenyl, 1,3-butadienyl, 2-pentenyl, isopropenyl, styryl, cinnamyl.

The term "alkynyl" refers to an univalent C₂ to C₁₂ and preferably C₂ to C₆ straight or branched hydrocarbon with at least one triple bond, optionally substituted with any suitable group, including but not limited to one or more moieties selected from lower alkyl or other groups as described above for the alkyl groups. Non-limiting examples are ethynyl, propynyl, 2-penten-4-yl, and -C≡C-CH₂-(alkyl) including -C≡C-CH₂CH₃.

The term "oxy derivatives", as used above includes -O-R⁴ groups wherein R⁴ is as defined below. Non-limiting examples are methoxy, ethoxy, phenoxy, benzyloxy, 2-naphtyloxy, 2-pyridyloxy, carbonate. R⁴ and R⁵ are the same or not and are independently selected from the group consisting of alkyl, alkenyl, alkynyl, heterocycle, aryl, arylalkyl. R⁴ and R⁵ can optionally be substituted with any suitable group, including but not limited to one or more moieties as described above for the alkyl groups.

The term "thio derivative" as used above, includes -S-R⁴ groups wherein R⁴ is defined above.

The term "amino derivatives" as used above, includes -NHR⁴ or -NR⁴R⁵ groups wherein R⁴ and R⁵ are defined above.

The term "aryl" as used above, includes an organic radical derived from an aromatic hydrocarbon by removal of one hydrogen, such as phenyl, biphenyl, naphthyl, which can optionally be substituted with any suitable group, including but not limited to one or more moieties selected from lower alkyl or other groups as described above for the alkyl groups. The aryl radical consists of 1-3 rings, preferably two rings, and contains 6-30 carbon atoms preferably 6-10 carbon atoms. Non-limiting examples are phenyl, biphenyl, cumenyl, mesityl, tolyl, xylyl, halophenyl, cyanophenyl, nitrophenyl, methoxyphenyl, naphthyl, indenyl, anthryl.

The term "halogen", as used above, includes an atom of Cl, Br, F, I.

The term "hydroxy", as used above, represents a group of the formula -OH.

The term "thiol", as used above, represents a group of the formula -SH.

The term "nitro", as used above, represents a group of the formula -NO₂.

The term "amino", as used above, represents a group of the formula -NH₂.

The term "carboxy", as used above, represents a group of the formula -COOH.

The term "heterocycle", as used above, refers to an aromatic or non aromatic cyclic alkyl, alkenyl, or alkynyl moiety as defined above, having at least one O, S and/or N atom interrupting the carbocyclic ring structure and optionally, one of the carbon of the carbocyclic ring structure may be replaced by a carbonyl. Non-limiting examples of aromatic heterocycles are pyridyl, furyl, pyrrolyl, thienyl, isothiazolyl, imidazolyl, benzimidazolyl, tetrazolyl, quinazolinyl, quinolizinyl, naphthyridinyl, pyridazinyl, pyrimidinyl, pyrazinyl, quinolyl, isoquinolyl, isobenzofuranyl, benzothienyl, pyrazolyl, indolyl, indolizinyl, purinyl, isoindolyl, carbazolyl, thiazolyl, 1,2,4-thiadiazolyl, thieno(2,3-b)furanyl, furopyranyl, benzofuranyl, benzoxepinyl, isooxazolyl, oxazolyl, thianthrenyl, benzothiazolyl, or benzoxazolyl, cinnolinyl, phthalazinyl, quinoxalinyl, phenanthridinyl, acridinyl, perimidinyl, phenanthrolinyl, phenothiazinyl, furazanyl, isochromanyl, indolinyl, xanthenyl, hypoxanthinyl, pteridinyl, 5-azacytidinyl, 5-azauracilyl, triazolopyridinyl, imidazolopyridinyl, pyrrolopyrimidinyl, and pyrazolopyrimidinyl optionally substituted as described above for the alkyl groups. Non-limiting examples of non aromatic heterocycles are tetrahydrofuranyl, tetrahydropyranyl, piperidinyl, piperidyl, piperazinyl, imidazolidinyl, morpholino , morpholinyl, 1-oxaspiro(4.5)dec-2-yl, pyrrolidinyl, 2-oxo-pyrrolidinyl, sugar moieties (i.e. glucose, pentose, hexose, ribose, fructose, which may also be substituted) or the same which can optionally be substituted with any suitable group, including but not limited to one or more moieties selected from lower alkyl, or other groups as described above for the alkyl groups. The term "heterocycle" also includes bicyclic, tricyclic and tetracyclic, spiro groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring or where a monocyclic heterocyclic group is bridged by an alkyl group, such as quinuclidinyl, 7-azabicyclo(2.2.1)heptanyl, 7-oxabicyclo(2.2.1)heptanyl, 8-azabicyclo(3.2.1)octanyl.

The term "arylalkyl", as used above, represents a group of the formula -L¹-aryl in which L¹ is as defined below and "aryl" as defined above. Non-limiting examples are benzyl, halobenzyl, cyanobenzyl, methoxybenzyl, (4,6-dimethoxybenzyl), nitrobenzyl, 2-phenylethyl, diphenylmethyl, di(4-ethoxyphenyl)methyl, di(4-methoxyphenyl)methyl, (4-methoxyphenyl)phenylmethyl, indenyl, anthracenylmethyl.

L¹ is selected from the group of C₁ to C₁₂ preferably C₁-C₅ straight or branched alkyl, alkylene or alkynylene groups.

The term "acyl derivative" as used above, represents a radical derived from carboxylic acid and thus includes groups of the formula R⁴-CO-, wherein R⁴ is defined above and may also be hydrogen. Non-limiting examples are formyl, acetyl, propionyl, isobutyryl, pivaloyl, valeryl, lauroyl, heptanedioyl, cyclohexanecarbonyl, crotonoyl, fumaroyl, acryloyl, benzoyl, naphthoyl, furoyl, nicotinoyl, 4-carboxybutanoyl, oxalyl, ethoxalyl, cysteinyl, oxamoyl.

The term "acyloxy derivatives" as used above, represents a radical of carboxylic acid and thus includes groups of the formula R⁴-CO-O-, wherein R⁴ is defined above and may also be hydrogen.

The term "ester" means a group of formula -COO-R⁴ wherein R⁴ is defined above.

The term "ether" means a group selected from C₁ to C₅₀ preferably C₁ to C₁₂ straight or branched alkyl, or C₂ to C₅₀ preferably C₂ to C₁₂ straight or branched alkenyl or alkynyl groups or a combination of the same, interrupted by one or more oxygen atoms.

The term "amido" means a group of formula -CONH₂ or -CONHR⁴ or -CONR⁴R⁵ wherein R⁴ and R⁵ are defined above.

The term "alkylene" refers to an alkyl moiety as described above in which a hydrogen atom has been removed to yield a divalent radical. Non-limiting example is -CH₂-CH(CH₃)-CH₂-.

The term "alkenylene" refers to an alkenyl moiety as described above in which a hydrogen atom has been removed to yield a divalent radical. Non-limiting example is -CH₂-CH=CH-CH₂-.

The term "alkynylene" refers to an alkynyl moiety as described above in which a hydrogen atom has been removed to yield a divalent radical. Non-limiting example is -C≡C-C(CH₃)₂-.

According to yet a more preferred embodiment, the present invention relates to the method of the invention as described above wherein in said compound of the general formula (I) R¹ represents Me(OR')CH wherein R' is hydrogen.

According to yet another more preferred embodiment, the present invention relates to the method of the invention as described above wherein in said compound of the general formula (I) R¹ represents Me(OR')CH wherein R' is R^{a}R^{b}R^{c}Si wherein R^{a}, R^{b} and R^{c} is methyl, ethyl, *i*-Pr, *t*-butyl or phenyl.

According to yet another more preferred embodiment, the present invention relates to the method of the invention as described above wherein in said compound of the general formula (I) R² represents an acyl derivative of the formula R"-CO- wherein R" is hydrogen, alkyl, substituted alkyl, preferably t-butyl, phenyl, substituted phenyl, OR^{d}, SR^{d} wherein R^{d} is C₁₋₆ alkyl, phenyl, substituted phenyl.

According to yet another more preferred embodiment, the present invention relates to the method of the invention as described above wherein in said compound of the general formula (I) R³ represents an arylalkyl, preferably methyl-bisphenyl or substituted methyl-bisphenyl. These substituents are preferred because the hydrogen of the methyl group in methyl-bisaryl groups can be easily abstract with a radical reagent. The resulting stabilized bis-aryl-methylene radical can be further quenched with a halogen radical.

According to yet another embodiment, said halogen radical source is provided from a N-halogeno-compounds which hemolytic cleavage furnishes a nitrogen radical and a halogen radical. The nitrogen radical abstracts the proton from the bis-phenyl-methyl group and the halogen radical traps the so-formed bis-phenyl-methylene radical. The cleavage of the N-halogeno reagent can be obtained by thermal activation, light activation, or activation with radical initiators such as AIBN (azo-bis-isobutyronitrile) and related azo compounds. Suitable N-halogeno reagents for use in the method of the invention are N-halogeno-amines, N-halogeno-amides, N-halogen-imides, N-halogeno-ureas, N-halogeno-carbamates, or N-halogeno-hydantoines.

According to a more preferred embodiment, said halogen radical source for use in the method of the invention as described above is N-bromosuccinimide (NBS) or N-bromophtalimide which are commercially available.

According to yet another aspect, the present invention relates to a method for the preparation of a compound of the general formula (I-A) wherein R' and R" are as noted above, Y and Y' are alkyl, alkyloxy, halogen or preferably hydrogen, wherein this method comprises the reaction of a compound of the general formula (II-B) in the presence of a halogen-containing nitrogen source, preferably N-bromosuccinimide (NBS), activated by light (hν) and in the presence of water and a proton donor as the catalyst, according to the following ***scheme 2.***

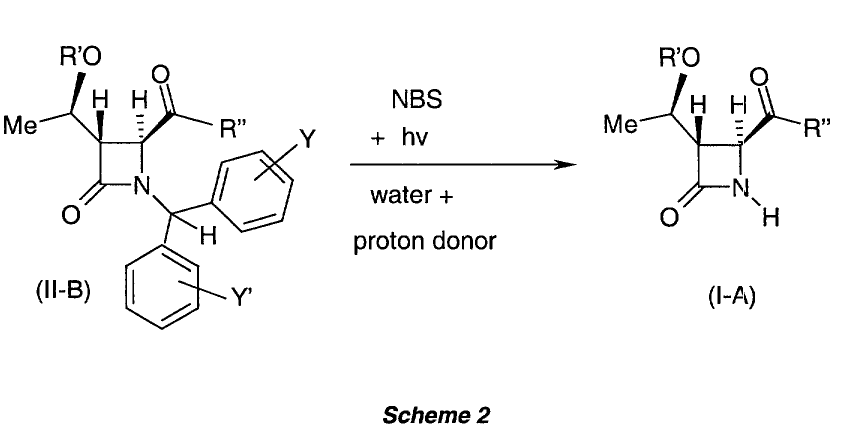

This method as described above converting compound of general formula (II-B) to (I-A) can be divided into three steps, as illustrated in the ***scheme 3*** below. In the first step, the compound of general formula (II-B) is halogenated by means of a halogen radical generated by the halogen-containing nitrogen source in the presence of light, forming a general compound of formula (II-C). In the presence of water said compound of general formula (II-C) is next converted into a compound of general formula (II-D). In the presence of a proton donor as the catalyst (an acid according to Brönsted), said compound of general formula (II-D) is converted into the compound of general formula (I-A) with expulsion of bis-aryl-ketone. Another advantage of the process according to the invention is that it can be performed in "one-pot" without isolation of intermediates, or with the isolation of intermediates II-D.

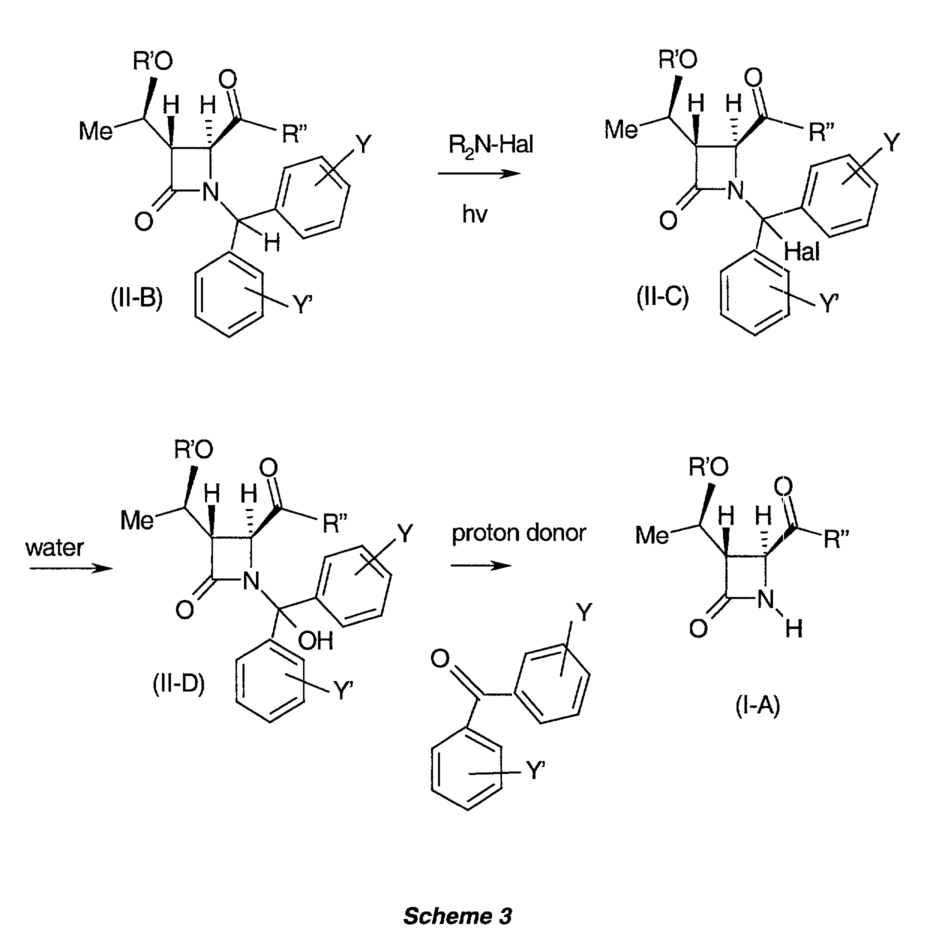

This present method of this invention, in particular removing the nitrogen-protecting group in the presence of a halogen-containing nitrogen source and light, and in the presence of water and a proton donor, have several advantages compared to the method currently known in the art. In particular, the method of this invention results in a higher production yield of azetidinones and makes use of environmentally acceptable conditions avoiding the utilization of large quantities of a metal such as Cerium IV.

Moreover, this method of N-deprotection can be generalized in all fields of organic synthesis, and more particularly in peptide synthesis where new orthogonal methods are useful.

According to yet another aspect, the present invention relates to a method for the preparation of a compound of the general formula (I-A) wherein R', R", Y and Y' are as noted above, wherein this method comprises the steps of
(a) converting L-threonine to sodium (2S,3R)-*cis*-2,3-epoxybutanoate,
(b) converting said sodium (2S,3R)-*cis*-2,3-epoxybutanoate to an epoxyamide of general formula (A)
(c) cyclization of said compound of general formula (A) into N-protected azetidinone (II-B), and
(d) removing the N-protecting group of the compound of the general formula (II-B) in the presence of a halogen-containing nitrogen source, preferably N-bromosuccinimide and light, and in the presence of water and a proton donor as the catalyst, according to the following ***scheme 4.***

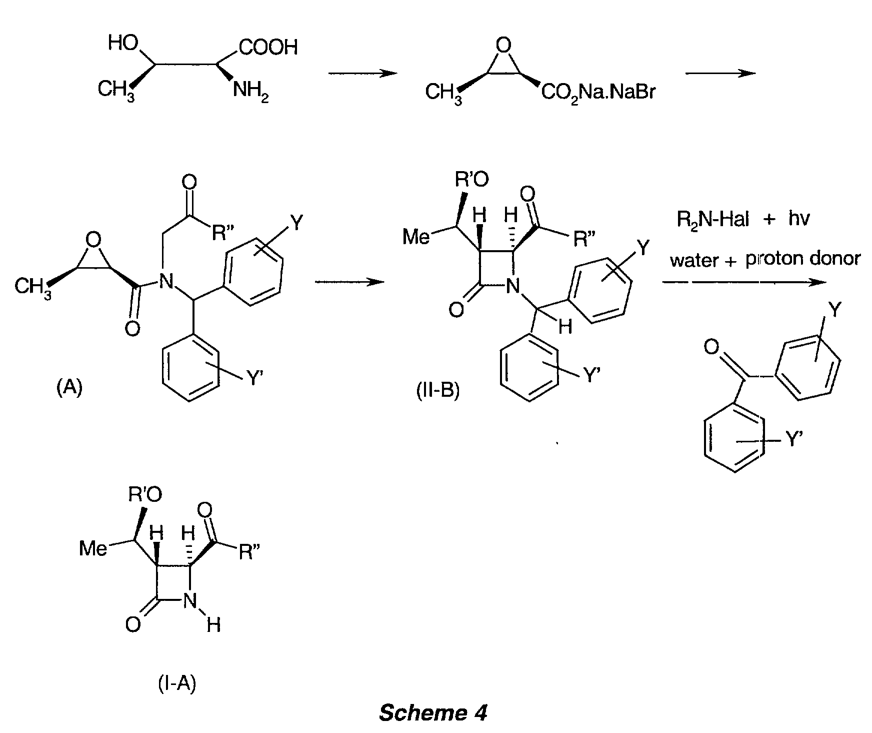

According to a more preferred embodiment, in the method of the invention as described above an extra step may optionally be present which comprises a silanating step for the protection of the hydroxyl function (R'= H), performed either before or after the deprotecting step (d).

The full process for the preparation of azetidinones starting from (2R,3R)-*cis*-2,3-epoxybutanoate according to the new process of this invention is illustrated in the following ***scheme 5*** and described hereafter.

Sodium (2R,3R)-*cis*-2,3-epoxybutanoate is prepared from L-threonine according to known procedures as described in Petit Y. et al., Synthesis 1988, 538 and Demillequand M. et al., J Chromatogr., A. 1999, 832, 259. Converting said (2R,3R)-*cis*-2,3-epoxybutanoate to an epoxyamide of the general formula (A) can be done by coupling to N-(bis-aryl-methyl)-N-(acyl-methyl) amine in several conditions of carboxyl activation such as SOCl₂/THF; SOCl₂ + pyridine/benzene; (COCl)₂ + pyridine/THF; 2,3,5-trichlorobenzoyl chloride/DMF; *i*-butyl chloroformate/DMF or CH₃CN; pivaloyl chloride + pyridine/CH₃CN. The next step consists of cyclization in the presence of a base such as K₂CO₃ in DMF (dimethylformamide) or LiHMDS (lithium hexamethyldisilazide) in benzene or THF (tetrahydrofuran) as described in Deng B.-L. et al. Tetrahedron, 2000, 56, 3209-3217. The N-protecting group is next removed according to the method of this invention as described above. The compound of the general formula (II-B) is reacted with N-halogenosuccinimide, for instance N-bromosuccinimide, in the presence of light. This reaction results in a compound of the general formula (II-C). In the presence of water, said compound of the general formula (II-C) is converted in the compound of the general formula (II-D), which is next converted into a compound of the general formula (I-A) by reaction with a proton donor as the catalyst. Said compound of the general formula (I-A) may next be silanated as illustrated in the last step of ***scheme 5****.* Optionally, silanation can be done before the deprotecting step.

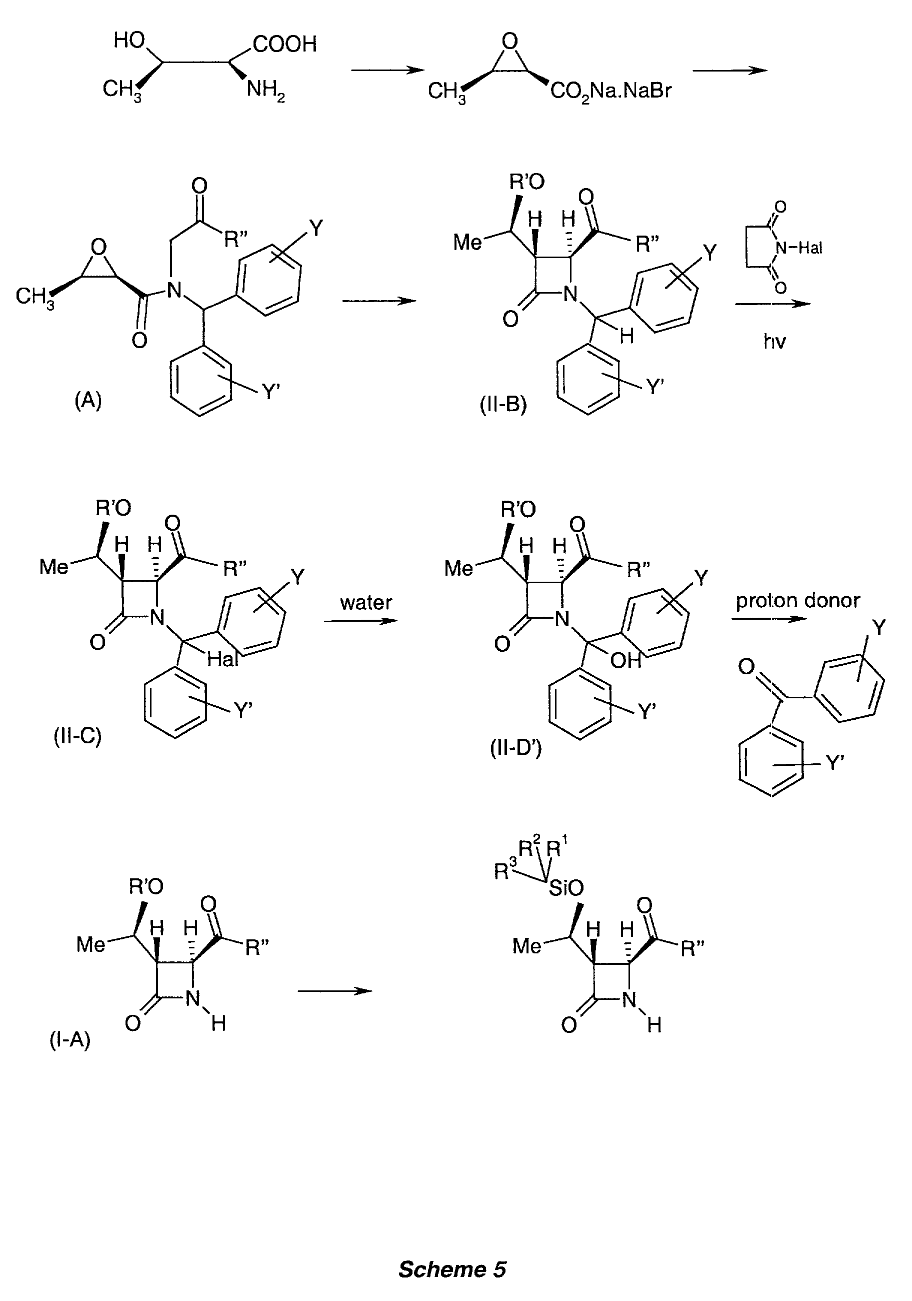

According to yet another embodiment, said proton donor in the method of the invention as described above is selected from the groups consisting of carboxylic acids (such as trichloroacetic acid, trifluoroacetic acid, formic acid,...), sulfonic acids (such as methane sulfonic acid, trifluoromethane sulfonic acid, para-toluene sulfonic acid,...) and inorganic acids (such as hydrochloric acid, sulfuric acid, perchloric acid, phosphoric acid,...).

According to yet another aspect, the present invention relates to compounds as a final product or as an intermediate obtainable by any of the methods of the invention as described above.

According to yet another aspect, the present invention also relates to a compound obtained by any of the methods of the invention as described above.

According to yet another aspect, the present invention also relates to a compound of the general formula (I-A) as noted above wherein R' is R^{a}R^{b}R^{c}Si wherein R^{a}, R^{b} and R^{c} are as noted above.

Furthermore, the invention also relates to pharmaceutically acceptable salts, geometrical isomers (including cis and trans, Z and E isomers), enantiomers, diastereoisomers and mixtures (including racemates) of the compounds of the invention as defined above.

The "pharmaceutically acceptable salts" according to the invention are meant to comprise the therapeutically active non-toxic base and acid salt forms which the compounds of the invention are able to form.

The acid salt form of a compound of the invention that occurs in its free form as a base can be obtained by treating said free base form with an appropriate acid such as an inorganic acid, for example, hydrogen halides e.g. hydrochloric or hydrobromic, sulfuric, nitric, phosphoric and the like acids; or an organic acid, such as, for example, acetic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like.

The compounds of the invention containing acidic protons may be converted into their therapeutically active non-toxic base, i.e. metal or amine, addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. N-methyl-D-glucamine, hydrabamine salts, and salts with amino acids such as, for example, arginine, lysine and the like.

Conversely said salt forms can be converted into the free forms by treatment with an appropriate base or acid.

This invention also relates to all stereoisomeric forms such as optical enantiomeric and diastereoisomeric forms of the compounds of formula I or mixtures (including racemates) thereof. The compounds of the invention and some of their intermediates have at least one stereogenic center in their structure. This stereogenic center may be present in a R or a S configuration, said R and S notation is used in correspondance with the rules described in Pure Appl. Chem., 45 (1976) 11-30.

Furthermore certain compounds of the invention which contain alkenyl groups may exist as Z or E isomers. In each instance, the invention includes both mixtures and separate individual isomers.

Some of the compounds of the invention may also exist in their tautomeric forms. Such forms although not explicity indicated in the above formula are intended to be included within the scope of the present invention.

The compound of the invention and its salt can be in the form of a solvate, which is included within the scope of the present invention. The solvate are for example hydrates, alcoholates and the like.

In the preparation methods according to the invention, the reaction products may be isolated from the reaction medium and, if necessary, further purified according to methodologies generally known in the art such as, for example extraction, crystallization, distillation, trituration and chromatography, or any combination of the same.

Pure stereochemically isomeric forms of said compounds of the invention (and said intermediates) can be obtained by the application of art-known procedures. For example, diastereoisomers can be separated by physical methods such as selective crystallization or chromatographic techniques, e.g. counter current distribution, liquid chromatography and the like methods.

Enantiomers can be obtained from racemic mixtures by first converting said racemic mixtures with suitable resolving agents such as, for example, chiral acids, to mixtures of diastereoisomeric salts or compounds; then physically separating said mixtures of diastereoisomeric salts or compounds by, for example, selective crystallization or chromatographic techniques, e.g. liquid chromatography and the like methods; and finally converting said separated diastereomeric salts or compounds into the corresponding enantiomers.

Alternatively, pure stereochemically isomeric forms may be obtained by using enantioselective reactions according to procedures known by the person skilled in the art.

Another alternative manner of separating the enantiomeric forms of the compounds of the invention and intermediates involves liquid chromatography, in particular liquid chromatography using a chiral stationary phase. Some of the intermediates are known compounds or may be prepared according to art-known procedures.

The following examples are provided for illustrative purposes only and are not intended, nor should they be construed, as limiting the invention in any manner. Those skilled in the art will appreciate that routine variations and modifications of the following examples can be made without exceeding the spirit or scope of the invention.

### EXAMPLES

### Example 1: Conversion of N-(benzhydryl)-N-(pivaloylmethyl) (2R, 3R)-cis-2,3-epoxybutyramide to (3S, 4S)-1-benzhydryl-3-[(5R)-1'-hydroxyethyl]-4-pivaloyl-2-azetidinone.

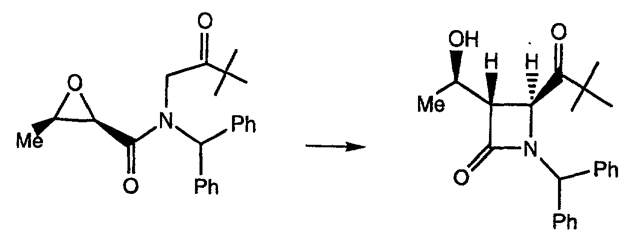

To a solution of N-(benzhydryl)-N-(pivaloylmethyl) (2*R*, 3*R*)-cis-2,3-epoxybutyramide (3.7 g, 10.13 mmol) in THF (70 mL), cooled at - 5° C, was added dropwise a solution of lithium hexamethyldisilazide (20 mL 1M LiHMDS) in THF (90 mL). The mixture was kept at - 5° C during the addition, then stirred at 0° C for 2 h. After addition of 1N HCl (75 mL), the mixture was extracted with ethyl acetate (2 x 50 mL). The organic phase was washed successively with 5 % NaHCO₃ (2 x 75 mL) and brine (2 x 75 mL), dried over MgSO₄ and concentrated under vacuum. Flash chromatography on silica gel (CH₂Cl₂ - EtOAc, 10 : 1; R_{F} = 0.15) gave (3*S*, 4*S*)-1-benzhydryl-3-[(5*R*)-1'-hydroxyethyl]-4-pivaloyl-2-azetidinone (3.33 g, 90 % yield) as a pale yellow oil which crystallized from ethanol, or ethyl acetate - hexane (1 : 4) : mp 168-170° C; IR (KBr) ν = 3500, 1759, 1704, 1457, 1367 cm⁻¹; ¹H NMR (500 MHz, CDCl₃) δ = 0.90 (s, 9H), 1.38 (d, 3H, *J* = 6.4 Hz), 2.04 (br s, 1H), 2.89 (dd, 1H, *J* = 2.1 and 6.4 Hz), 4.21 (dq, 1H, *J* = 6.4 and 6.4 Hz), 4.52 (d, 1H, *J* = 2.1 Hz), 5.69 (s, 1H), 7.20 - 7.40 (m, 10H); HRMS : 365.1988 (calcd for C₂₃H₂₇NO₃ : 365.1991).

### Example 2: Conversion of N-(Benzhydryl)-N-(phenacyl) (2R, 3R)-cis-2,3-epoxybutyramide to (3S, 4S)-1-benzhydryl-3-[(5R)-1'-hydroxyethyl]-4-benzoyl-2-azetidinone.

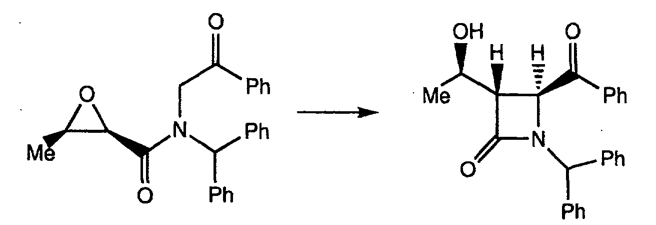

N-(Benzhydryl)-N-(phenacyl) (2*R*, 3*R*)-*cis*-2,3-epoxybutyramide (1.04 g, 2.7 mmol) was reacted with LiHMDS as described in example 1, to furnish (3*S*, 4*S*)-1-benzhydryl-3-[(5*R*)-1'-hydroxyethyl]-4-benzoyl-2-azetidinone (0.593 g, 57 % yield) as a white gum : IR (KBr) δ = 3400, 1741, 1685, 1450 cm⁻¹; ¹H NMR (500 MHz, CDCl₃) ν = 1.20 (d, 3H, *J*= 6.3 Hz), 2.10 (br, s, 1H), 3.11 (dd, 1H, *J* = 2.3 and 5.2 Hz), 4.31 (dq, 1H, *J* = 5.2 and 6.3 Hz), 5.07 (d, 1H, *J* = 2.3 Hz), 5.59 (s, 1H), 7.15 - 7.40 (m, 12H), 7.50 (t, 1H, *J* = 7.7 Hz), 7.70 (d, 2H, *J* = 7.7 Hz); HRMS : 385.1692 (calcd for C₂₅H₂₃NO₃ : 385.1678).

### Example 3: Conversion of N-(4,4'-Dimethoxybenzhydryl)-N-(plvaloylmethyl) (2R, 3R)-cis-2,3-epoxybutyramide to (3S, 4S)-1-(4,4'-dimethoxybenzhydryl)-3-[(5R)-1'-hydroxyethyl]-4-pivaloyl-2-azetidinone.

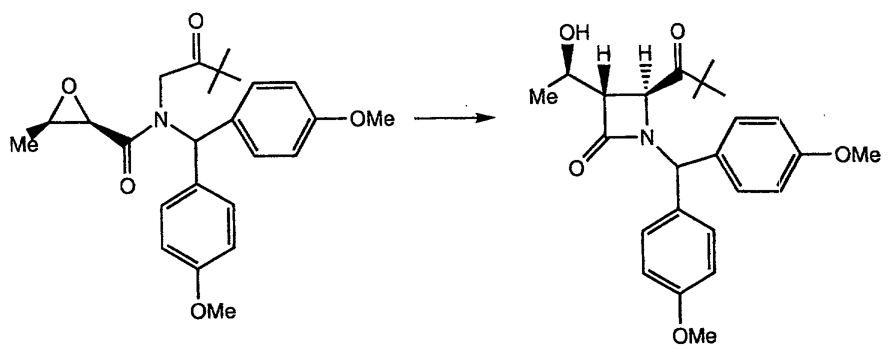

N-(4,4'-Dimethoxybenzhydryl)-N-(pivaloylmethyl) (2*R*, 3*R*)-*cis*-2,3-epoxybutyramide (0.34 g, 0.80 mmol) was reacted with LiHMDS as described in example 1, to furnish (3*S*, 4*S*)-1 -(4,4'-dimethoxybenzhydryl)-3-[(5R)-1 '-hydroxyethyl]-4-pivaloyl-2-azetidinone (0.21 g, 60 % yield) as a white solid: IR (KBr) : 2985, 1786, 1683, 1612 cm⁻¹; ¹H NMR (200 MHz, CDCl₃) : 0.93 (s, 9H), 1.36 (d, 3H, *J* = 6.3 Hz), 2.03 (br s, 1H), 2.87 (dd, 1H, *J* = 2.1 and 5.9 Hz), 3.76 and 3.78 (s, 6H), 4.21 (dq, 1H, *J* = 5.9 and 6.3 Hz), 4.50 (d, 1H, *J* = 2.1 Hz), 5.57 (s, 1H), 6.80-6.88 (m, 4H), 7.16 - 7.27 (m, 4H). Anal. calcd for C₂₅H₃₁O₅N : C, 70.57; H, 7.34; N, 3.29- Found : C, 70.12; H, 7.15; N, 3.58.

### Example 4 : Conversion of (3S,4S)-1-benzhydryl-3-[(5R)-1'-hydroxyethyl]-4-pivaloyl-2-azetidinone to (3S, 4S)-1-benzhydryl-3-[(5R)-1'-t-butyldimethylsilyloxyethyl]-4-pivaloyl-2-azetidinone.

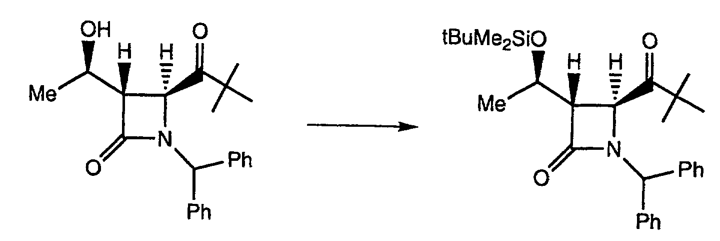

*t*-Butyldimethylsilyl chloride (2.65 g, 18 mmol) and imidazole (2.15 g, 32 mmol) were added to a solution of (3*S*, 4*S*)-1 -benzhydryl-3-[(5*R*)-1'-hydroxyethyl]-4-pivaloyl-2-azetidinone (1.03 g, 2.82 mmol) in DMF (50 mL). The mixture was stirred, under argon atmosphere, at room temperature, during 48 h, then diluted with ethyl acetate (50 mL). The organic phase was washed successively with water (2 x 50 mL) and brine (2 x 50 mL). Drying over MgSO₄, concentration and flash chromatography on silica gel (elution with CH₂Cl₂; R_{f} = 0.08) gave (3*S*, 4*S*)-1-benzhydryl-3-[(5*R*)-1'-*t*-butyldimethylsilyloxyethyl]-4-pivaloyl-2-azetidinone as a colourless oil (1.34 g, 93 % yield) : IR (film) ν = 1768, 1710cm¹; ¹H NMR (CDCI3, 200 MHz) δ=0.13 (s, 6H), 0.87 (s, 9H), 0.93 (s, 9H), 1.30 (d 3H, *J* = 6.3 Hz), 2.82 (dd, 1H, *J* = 1.9 and 5.5 Hz), 4.27 (dq, 1H, *J* = 5.5 and 6.3 Hz), 4.58 (d, 1H, *J* = 1.9 Hz), 5.68 (s, 1H), 7.2 - 7.4 (m, 10H); Anal. calcd for C₂₉H₄₁O₃NSi : C, 72.61; H, 8.61; N, 2.92 - Found : C, 72.58; H, 8.71; N, 2.88.

### Example 5: Conversion of (3S, 4S)-1-benzhydryl-3-[(5R)-1'-hydroxyethyl]-4-pivaloyl-2-azetidinone to (3S, 4S)-1-(diphenyl)hydroxymethyl-3-[(5R)-1'-hydroxyethyl]-4-pivaloyl-2-azetidinone.

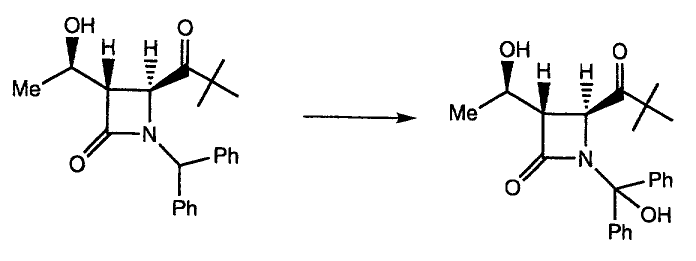

A solution of (3*S*, 4*S*)-1-benzhydryl-3-[(5*R*)-1'-hydroxyethyl]-4-pivaloyl-2-azetidinone (0.52 g, 1.4 mmol) and N-bromosuccinimide (NBS) (0.32 g, 1.8 mmol) in chlorobenzene (50 mL) and water (5 mL) was stirred at room temperature, overnight, under the irradiation of the fume hood's lamp. After dilution with CH₂Cl₂ (50 mL), the organic phase was washed with 5 % aqueous NaHSO₃ (100 mL), then concentrated under vacuum at 20° C to give crude (3*S*, 4*S*)-1-(diphenyl)hydroxymethyl-3-[(5*R*)-1'-hydroxyethyl]-4-pivaloyl-2-azetidinone as a pale yellow solid (0.53 g, 100 % yield) : ¹H NMR (200 MHz, CDCl₃) δ = 0.84 (s, 9H), 1.43 (d, 3H, J = 6.6 Hz), 2.50 (br s, 1H), 2.87 (dd, 1H, *J* = 2.1 and 6.3 Hz), 4.26 (dq, 1H, *J* = 6.3 and 6.3 Hz), 4.69 (d, 1H, *J* = 2.1 Hz), 5.12 (br s, 1H), 7.2 - 7.8 (m, 10H).

### Example 6: Conversion of (3S, 4S)-1-(diphenyl)hydroxymethyl-3 [(5R)-1'-hydroxyethyl]-4-pivaloyl-2-azetidinone to (3S, 4S)-3-[(5R)-1'-hydroxyethyl]-4-pivaloyl-2-azetidinone.

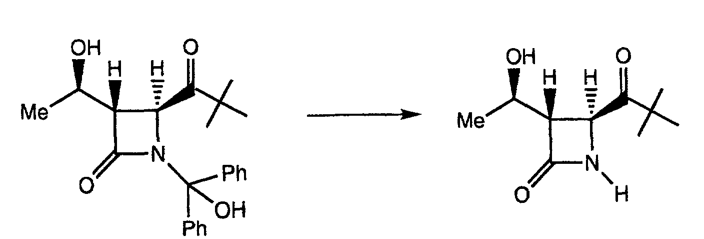

A solution of (3*S*, 4*S*)-1-(diphenyl)hydroxymethyl-3-[(5*R*)-1'-hydroxyethyl]-4-pivaloyl-2-azetidinone (0.53 g, 1.4 mmol) and *p*-toluenesulfonic acid (30 mg, 0.16 mmol) in acetonitrile-water (1 : 1, v/v; 20 mL) was stirred overnight at room temperature. After dilution with ethyl acetate (20 mL), the organic phase was washed with 5 % aqueous NaHCO₃ (30 mL), then concentrated under vacuum and purified by flash chromatography on silica gel (elution with CH₂Cl₂ : EtOAc, 10 : 1, then with EtOAc; R_{f} = 0.52) to give (3*S*, 4*S*)-3-[(5*R*)-1'-hydroxyethyl]-4-pivaloyl-2-azetidinone as a white solid (0.265 g, 95 % yield): ¹H NMR (CDCl₃, 200 MHz) δ = 1.23 (s, 9H), 1.36 (d, 3H, *J* = 6.3 Hz), 2.3 (br s, 1H), 3.21 (dd, 1H, *J* = 2.2 and 6.3 Hz), 4.24 (dq, 1H, *J* = 6.3 and 6.3 Hz), 4.58 (d, 1H, *J* = 2.2 Hz), 6.47 (s, 1H); ¹³C NMR (CDCl₃, 50 MHz) ν = 22.0, 26.6, 44.3, 52.6, 64.1, 65.9, 169.0, 213.0; Anal. calcd for C₁₀H₁₇O₃N : C, 60.28; H, 8.60; N, 7.03 - Found : C, 60.13; H, 8.70; N, 6.96 %.

### Example 7: Conversion of (3S, 4S)-1-benzyhydryl-3-[(5R)-1'-(t-butyldimethylsilyloxy)ethyl]-4-pivaloyl-2-azetidinone to (3S, 4S)-1-(diphenyl)hydroxymethyl-3-[(5R)-1'-(t-butyldimethylsilyloxy)ethyl]-4-pivaloyl-2-azetidinone.

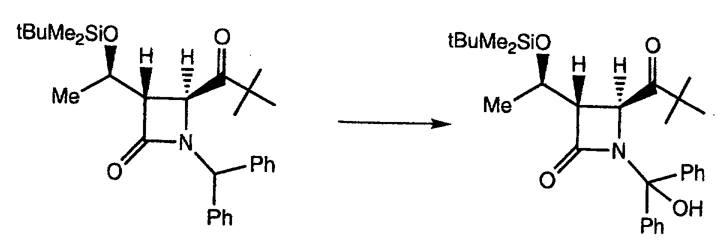

A solution of (3*S*, 4*S*)-1-benzyhydryl-3-[(5*R*)-1'-(*t*-butyldimethylsilyloxy)ethyl]-4-pivaloyl-2-azetidinone (0.13 g, 0.27 mmol) in wet chlorobenzene was treated with NBS under irradiation, as described in example 5, to furnish crude (3*S*, 4*S*)-1-(diphenyl)hydroxymethyl-3-[(5*R*)-1'-(*t*-butyldimethylsilyloxy)ethyl]-4-pivaloyl-2-azetidinone as a pale yellow solid (0.11 g, 81% yield) : ¹H NMR (CDCI3, 200 MHz) δ= 0.15 (s, 3H), 0.16 (s, 3H), 0.80 (s, 9H), 0.96 (s, 9H), 1.33 (d, 3h, J=6.3 Hz), 2.78 (dd, 1H, J=6 and 2.2 Hz), 4.30 (m, 1H), 4.72 (d, 1H, J=2.2 Hz), 5.18 (brs, 1H), 7.1-7.6 (m, 10H).

### Example 8: Conversion of (3S, 4S)-1-(diphenyl)hydroxymethyl-3-[(5R)-1'-(t-butyldimethylsilyloxy) ethyl]-4-pivaloyl-2-azetidinone to (3S, 4S)-3-[(5R)-1'-(t-butyldimethylsilyloxy)ethyl]-4-pivaloyl-2-azetidinone.

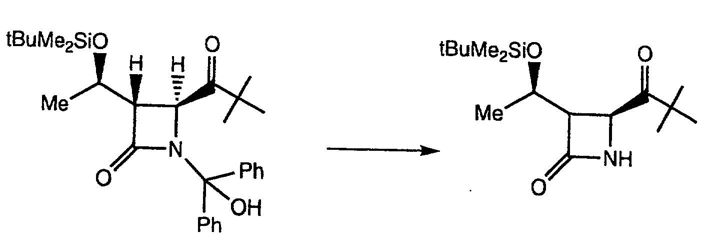

A solution of (3*S*, 4*S*)-1-(diphenyl)hydroxymethyl-3-[(5*R*)-1'-(*t*-butyldimethylsilyloxy) ethyl]-4-pivaloyl-2-azetidinone (0.11g, 0.22 mmol) in CH₃CN-H₂O was treated as described in example 6, to furnish (3*S*, 4*S*)-3-[(5*R*)-1'-(*t*-butyldimethylsilyloxy)ethyl]-4-pivaloyl-2-azetidinone ( 0.066 g, 95% yield). This compound was identical to the product obtained in example 9.

### Example 9: Conversion of (3S, 4S)-3-[(5R)-1'-hydroxyethyl]-4-pivaloyl-2-azetidinone to (3S, 4S)-3-[(5R)-1'-(t-butyldimethylsilyloxy)ethyl-4-pivaloyl-2-azetidinone.

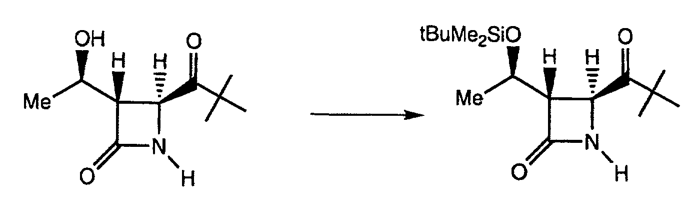

*t*-Butyldimethylsilyl chloride (2.04 g, 14 mmol) and imidazole (2.31 g, 34 mmol) were added to a solution of (3*S*, 4S)-3-[(5*R*)-1'-hydroxyethyl]-4-pivaloyl-2-azetidinone (1.35 g, 6.8 mmol) in DMF (70 ml). The mixture was stirred, under argon atmosphere, at room temperature, during 16 h, then diluted with ethyl acetate (50 mL). The organic phase was washed with brine (3 x 50 mL). Drying over MgSO₄, concentration and flash chromatography on silica gel (elution with CH₂Cl₂ : EtOAc, 10 : 1; R_{f} = 0.36) gave (3*S*, 4*S*)-3-[(5*R*)-1'-(*t*-butyldimethylsilyloxy)ethyl-4-pivaloyl-2-azetidinone as a white solid (1.47 g, 70 % yield): IR (KBr) ν = 3158, 2954, 1767, 1723, 1472 cm⁻¹; ¹H NMR (CDCl₃, 300 MHz) δ = 0.09 and 0.10 (s, 6H), 0.89 (s, 9H), 1.22 (s, 9H), 1.26 (d, 3H, *J* = 6.3 Hz), 3.17 (m, 1H), 4.27 (dq, 1H, *J* = 6.3 and 4.5 Hz), 4.59 (d, 1H, *J* = 2.1 Hz), 5.96 (br s, 1H).

### Example 10: Conversion of (3S, 4S)-1-benzhydryl-3-[(5R)-1'-hydroxyethyl]-4-benzoyl-2-azetidinone to (3S,4S)-3-[(5R)-1'-hydroxyethyl]-4-benzoyl-2-azetidinone

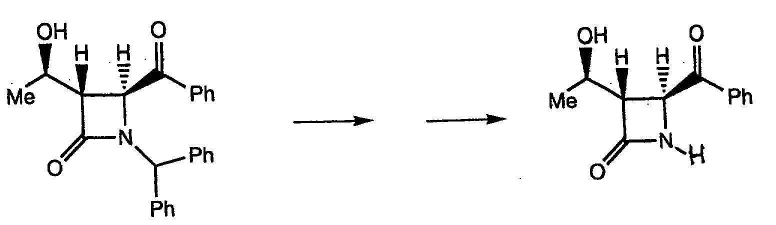

A solution of (3S, 4S)-1-benzhydryl-3-[(5R)-1'-(t-butyldimethylsilyloxy)ethyl]-4-benzoyl-2-azetidinone (0.11 g, 0.28 mmol) in wet chlorobenzene was treated with NBS under light, as described in example 5, to furnish crude (3S, 4S)-1-diphenylhydroxymethyl-3-[(5R)-1'-(t-butyldimethylsilyloxy)ethyl]-4-benzoyl-2-azetidinone as a pale yellow oil. The product is then treated as described in example 6 in CH₃CN-H₂O with *p*-Tos OH to furnish (3S, 4S)-3-[(5R)-1'-(t-butyldimethylsilyloxy)ethyl]-4-benzoyl-2-azetidinone (0.059 g, 95%).
¹H NMR : 1.34 (3H, d, 6.2 Hz); 3.25 (1H, dd, 2.6 Hz, *1.2Hz*); 4.30 (1H, m, 6.4 Hz); 5.07(1 H, d, 2.6 Hz); 7.4-7.8 (3H, m); 8.11 (2H, d, 7.2 Hz).

## Claims

1. A method for the preparation of a compound of the general formula (I) wherein
R¹ and R² each represent independently hydrogen, halogen, hydroxy, thiol, nitro, amino, carboxy, amino derivative, oxy derivative, thio derivative, alkyl, alkenyl, alkynyl, aryl, oxy derivative, amino derivative, thiol derivative, acyl derivative, acyloxy derivative, ester, amido, ether, arylalkyl, heterocycle or Me(OR')CH wherein R' is hydrogen or R^{a}R^{b}R^{c}Si wherein R^{a}, R^{b} and R^{c} is independently chosen from the group consisting of alkyl, alkenyl, alkynyl, aryl or a heterocycle;
R³ represents a protecting group such as alkyl, alkenyl, alkynyl, or arylalkyl;
wherein this method comprises the reaction of a compound of the general formula (II) in the presence of a halogen-radical source, water and a proton donor suitable as a catalyst, according to the following scheme.

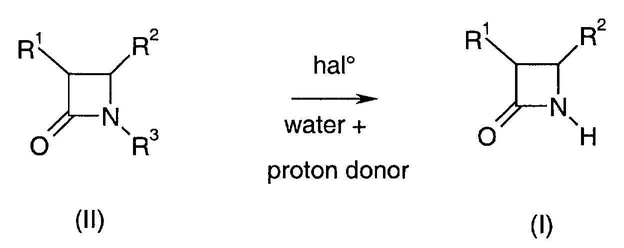

2. A method according to claim 1 wherein R¹ represents Me(OR')CH wherein R' is hydrogen.

3. A method according to claim 1 wherein R¹ represents Me(OR')CH wherein R' is R^{a}R^{b}R^{c}Si wherein R^{a}, R^{b} and R^{c} independently represent methyl, ethyl, *i*-Pr, *t*-butyl or phenyl.

4. A method according to claims 1-3 wherein R² represents an acyl derivative of the formula R"-CO- wherein R" is chosen from the group consisting of hydrogen, alkyl, substituted alkyl, preferably t-butyl, phenyl, substituted phenyl, OR^{d} or SR^{d} wherein R^{d} is C₁₋₆ alkyl, substituted alkyl, phenyl, substituted phenyl.

5. A method according to claims 1-4 wherein R³ represents an arylalkyl, preferably methyl-bisphenyl or substituted methyl-bisphenyl.

6. A method according to claims 1-5 wherein said halogen radical is provided from a N-halogeno compound selected from the group consisting of N-halogeno-amines, N-halogeno-amides, N-halogeno-imides, N-halogeno-ureas, N-halogeno-carbamates or N-halogeno-hydantoines.

7. A method according to claim 5 wherein said halogen radical source is N-halogenosuccinimide, preferably N-bromosuccinimide.

8. A method according to any of the claims 1-7 for the preparation of a compound of the general formula (I-A) wherein
R' is as noted in claims 2 or 3, R" is as noted in claim 4, and Y and Y' are alkyl, alkyloxy, halogen or preferably hydrogen,
comprising the reaction of a compound of the general formula (II-B) in the presence of a halogen radical source, preferably N-bromosuccinimide (NBS) activated by light, and in the presence of water and a proton donor suitable as a catalyst, according to the following scheme.

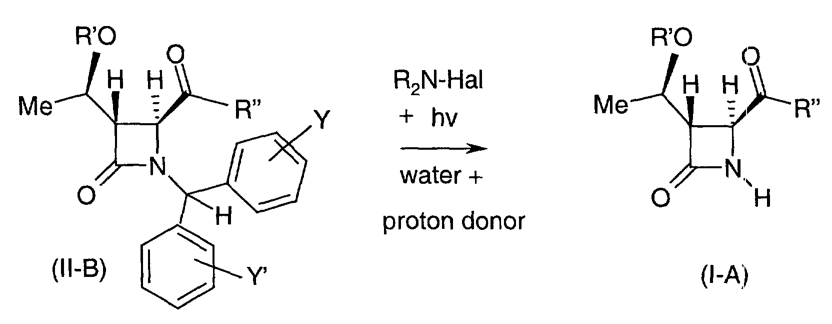

9. A method according to claim 8 for the preparation of a compound of the general formula (I-A) comprising the steps of
(a) converting L-threonine to sodium (2S,3R)-*cis*-2,3-epoxybutanoate
(b) converting said sodium (2S,3R)-*cis*-2,3-epoxybutanoate to an epoxyamide of general formula (A),
(c) cyclization of said compound of general formula (A), into azetidinone (II-B) and
(d) removing the N-protecting group of the compound of the general formula (II-B) in the presence of a halogen radical source, preferably a halogen-containing nitrogen source, and more preferably N-bromosuccinimide and light, in the presence of water and a proton donor suitable as a catalyst, according to the following scheme:

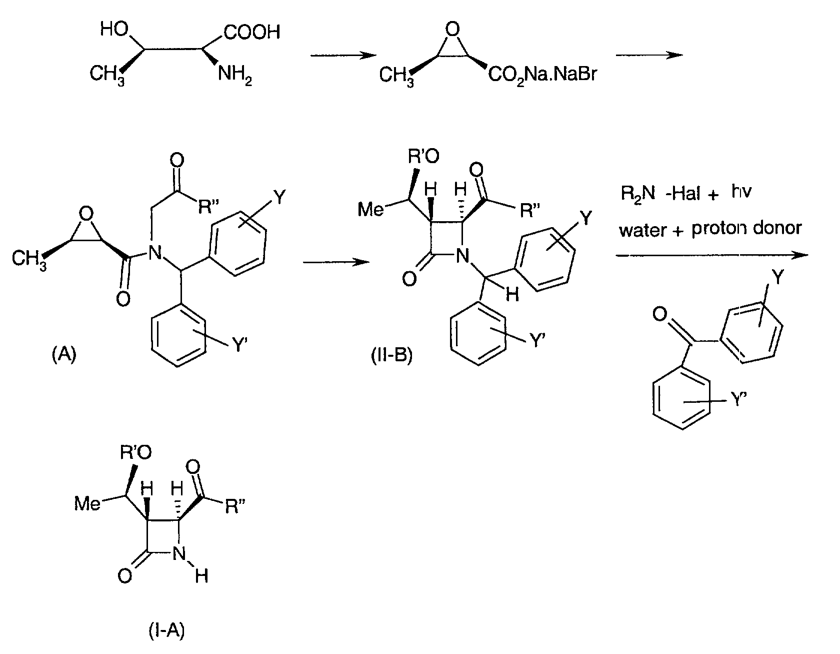

10. A method according to claim 9 further optionally comprising a silanating step for the protection of the hydroxyl function when R' is H performed either before or after the deprotecting step (d).

11. A method according to any of claims 1-10 wherein said proton donor is selected from the group consisting of carboxylic acids, sulfonic acids and inorganic acids.

12. A compound or intermediate obtainable by any of the methods according to claims 1-11.

13. A compound or intermediate obtained by any of the methods according to claims 1-11.

14. A compound of the general formula (I-A) wherein R" is *t*-butyl.

15. A compound of the general formula (I-A) wherein R' is R^{a}R^{b}R^{c}Si wherein R^{a}, R^{b} and R^{c} are as noted as in claim 3.

16. A pharmaceutically acceptable salt, geometrical isomer (including cis and trans, Z and E isomers), enantiomer, diastereoisomer or mixture (including racemates) of any compound according to any of claims 12-15.

17. Use of the method according to any of the claims 1-11 as an N-deprotection step in organic synthesis, and more preferably in peptide synthesis.
